# EUROPEAN PATENT SPECIFICATION

(11) **EP 1 795 573 B1**
(45) Date of publication and mention of the grant of the patent: **05.08.2009**
(21) Application number: 06024732.7
(22) Date of filing: 29.11.2006
(51) Int. Cl.: C09K 11/08, G01N 33/52

(54) **Semiconductor nanoparticle surface modification method**
Verfahren zur Modifizierung der Oberfläche von Halbleiter-Nanopartikeln
Méthode pour la modification de la surface des nanoparticules semiconductrices

(30) Priority: 06.12.2005 JP 2005351558
(43) Date of publication of application: 13.06.2007
(73) Proprietor: HITACHI SOFTWARE ENGINEERING CO., LTD., Yokohama-shi, Kanagawa 230-0045 (JP)
(72) Inventor: Sato, Keiichi, Shinagawa-ku Tokyo 140-0002 (JP); Hattori, Shinya, Shinagawa-ku Tokyo 140-0002 (JP); Chiba, Taeko, Shinagawa-ku Tokyo 140-0002 (JP)
(74) Representative: Liesegang, Eva

(56) References cited:
- EP-A- 1 679 359
- EP-A1- 1 333 280
- EP-A2- 1 514 909
- US-A1- 2002 045 045
- US-A1- 2003 194 731

## Description

### BACKGROUND OF THE INVENTION

### Field of the Invention

The present invention relates to a semiconductor nanoparticle having high luminescence properties and a method for synthesizing the same. Moreover, the present invention relates to a fluorescent reagent and an optical device comprising such semiconductor nanoparticle.

### Background Art

Semiconductor nanoparticles with particle sizes of 10 nm or less are located in the transition region between bulk semiconductor crystals and molecules, so that they exhibit physicochemical properties different from those of either bulk semiconductor crystals or molecules. In such region, the degeneracy of energy bands that is observed in bulk semiconductors is removed and the orbits become discrete, so that a quantum size effect appears in which the energy width of the forbidden band changes depending on particle size. According to the appearance of the quantum size effect, the energy width of the forbidden band of a semiconductor nanoparticle decreases or increases in response to an increase or decrease of particle size. This change of the energy width of the forbidden band affects the fluorescence properties of the particle in question. A particle that has a smaller particle size and wider forbidden band energy width tends to have a shorter a fluorescent wavelength, while a particle that has a larger particle size and a narrower forbidden band energy width tends to have a longer fluorescent wavelength. That is, it is possible to create a desired fluorescent color by controlling particle size. In addition to the properties described above, semiconductor nanoparticles have high durability against excitation lights, etc., and a region which can be excited widely extends more towards the shorter wavelengths than the fluorescent wavelength, so that simultaneous excitation of multiple fluorescent colors is also possible by using a single excitation light source. Thus, semiconductor nanoparticles serving as fluorescent material are gaining significant attention. Specifically, the fields related to biotechnology and to optical device technology are listed as fields in which semiconductor nanoparticles have been used actively, and further applications are expected in the future.

In order to use semiconductor nanoparticles serving as fluorescence material, it is desired that such particles have fluorescence properties in which the fluorescence spectrum has a waveform with a narrow and sharp full width half maximum (FWHM). Thus, it is necessary that the band gap fluorescence properties in response to the forbidden band widths of the semiconductor nanoparticles are made effective. However, even if a prepared bulk particle has a monodisperse particle size, such particle per se does not exhibit sufficient band gap fluorescence properties. As a reason for this, the presence of the energy level existing mainly at the surface site of the semiconductor nanoparticle is mentioned, and, since the energy level exists in the forbidden band inside the particle, it has been thought that the band gap fluorescence properties are inhibited. Due to the reasons mentioned above, the inactivation of the aforementioned energy level and the obtaining of the band gap fluorescence have become significant subjects.

A method for providing a solution to this subject relates to a (CdSe)ZnS semiconductor nanoparticle, which has a so-called core-shell type structure. The aforementioned method involves obtaining high luminescence properties by coating the semiconductor nanoparticle (CdSe) with a second semiconductor material (ZnS), which has a wider forbidden band width than that of the particle, and removing the energy level in the forbidden band of the particle, thereby making the band gap fluorescence properties effective. (JP Patent Publication (Kohyo) No. 2001-523758 A and J. Phys. Chem. B. 101:9463 (1997))

EP 1 679 359 A1 discloses semiconductor nanoparticles modified with functional group-containing polymers that electrostatically bind to the surface of the semiconductor nanoparticles. Octadecylamine is not disclosed as semiconductor nanoparticle-coating compound.

US 2002/0045045 A1 discloses water-dispersible nanoparticles coated with a multiply amphipathic dispersant and a method for the production of such nanoparticles. Electrostatic interactions between a functional group-containing polymer and octadecylamine as nanoparticle-coating compound are not disclosed.

In addition, by achieving particle size monodispersion in an aqueous solution and carrying out particle surface reforming, inventors have been studying a method for making band gap fluorescence effective. As a result of intensive studies carried out by the inventors, a method for obtaining semiconductor particles having commercially adequate fluorescence properties has been developed, in which semiconductor nanoparticles synthesized by a size-selective photoetching technique are treated in a refining process, the particles are subjected to surface reforming using sodium hydroxide or amine-ammonium compounds, and the energy levels at the particle surfaces are made inactive by arranging the electron-releasing groups on the surfaces, such that the band gap fluorescence properties are made effective. Moreover, by coating the obtained nanoparticles with organic compounds such as one composed of amphiphilic molecules, we succeeded in obtaining semiconductor nanoparticles having improved chemical durability. According to a series of these methods, synthesis of semiconductor nanoparticles that have high luminescence properties was realized by using a safe and simple technique in an aqueous solution. The nanoparticles per se have sufficient durability. In addition, high durability can be imparted to them by allowing preferably usable organic compounds, such as amphipathic molecules, to bind to each other. However, for the purpose of synthesizing high-functional semiconductor nanoparticles by a more convenient method, the inventors have arrived at the present invention.

### SUMMARY OF THE INVENTION

The inventors have invented a surface treatment, such as an OH coating or ammonia treatment, as a surface reforming technique for semiconductor nanoparticles. However, semiconductor nanoparticles that have been subjected to a surface treatment, such as an OH coating or ammonia treatment, do not have sufficient durability against external factors, typically including pH. It has been an objective to solve the aforementioned problems.

In order to protect semiconductor nanoparticles from the aforementioned external factors, the inventors have attempted a method of coating obtained nanoparticles with organic material. Further, the inventors have conducted studies of semiconductor nanoparticles having chemical durability and showing high luminescence properties. When semiconductor nanoparticles are utilized for bio-related applications, it is preferable to modify the surfaces of such semiconductor nanoparticles with a functional group such as a carboxyl group. Also, it is necessary to modify semiconductor nanoparticles so as to improve the industrial availability thereof.

The inventors found that semiconductor nanoparticles exerting high luminescence properties can be obtained by applying surface reforming to semiconductor nanoparticles, and modifying the semiconductor nanoparticles with a functional group-containing polymer, thereby allowing the polymer to form a crosslink via a crosslinking agent.

That is, firstly, the present invention is an invention of a semiconductor nanoparticle exerting high luminescence properties, which is modified with a functional group-containing polymer that electrostatically binds to the semiconductor nanoparticle as defined in the claims.

Preferably, electron-releasing groups are arranged on the surface of the semiconductor nanoparticle, and the polymer electrostatically binds to the outside of the electron-releasing groups. Preferably, the functional group-containing polymer electrostatically binds to the surface of the semiconductor nanoparticle, and the modifying groups of the functional group-containing polymer form a crosslinking bond via a crosslinking agent.

Preferably, specific examples of functional groups of the functional group-containing polymer include, but are not limited to, one or more functional groups selected from the group consisting of -COOH, -OH, -NH₂, -SH, -OCN, -CNO, -CHO, -CH=O, -CH=CH₂, and -C≡CH, so that various types of crosslinking reactions can be involved.

The functional group-containing polymer may directly bind to the surface of a semiconductor nanoparticle or bind thereto via a semiconductor nanoparticle-coating compound.

Preferably, specific examples of the crosslinking bond include one or more bonds selected from the group consisting of an ester bond, an amide bond, an imide bond, an ether bond, a urethane bond, a sulfide bond, a polysulfide bond, a carbonate bond, a thiol bond, a thioester bond, and a thiourethane bond. The crosslinking bond comprises a crosslink that results from carbon-carbon double bond or carbon-carbon triple bond polymerization.

In preferred examples, the functional group-containing polymer is polyacrylic acid and the crosslinking agent is alkylene diamine.

Preferably, examples of the electron-releasing group include at least one group selected from the group consisting of -OR, -OCH₂R, -OCOCH₂R, -NHR, -N(CH₂R)₂, -NHCOCH₂R, -CH₂R, and -C₆H₄R, where R is hydrogen, a substituted hydrocarbon group, or an unsubstituted hydrogen group. In addition, the semiconductor nanoparticle surface-coating compound is an C₈₋₂₄ alkyl amine namely an octadecylamine.

Preferably, specific examples of the semiconductor nanoparticle material include, but are not limited to, one or more materials selected from the group consisting of ZnO, ZnS, ZnSe, ZnTe, CdO, CdS, CdMnS, CdSe, CdMnSe, CdTe, CdMnTe, HgS, HgSe, HgTe, InP, InAs, InSb, InN, GaN, GaP, GaAs, GaSb, TiO₂, WO₃, PbS, PbSe, MgTe, AlAs, AlP, AlSb, AIS, Ge, and Si. In addition, a semiconductor nanoparticle having a multilayer structure consisting of a core portion and a shell portion may be made of one or more member of the aforementioned group.

The particle size of the semiconductor nanoparticle of the present invention exhibits a deviation of less than 10% rms in diameter, thereby achieving monodispersion.

Moreover, the semiconductor nanoparticle of the present invention is characterized in that it emits light in a narrow spectrum range of less than 60 nm in terms of full width at half maximum (FWHM) upon being irradiated with excitation light.

Secondly, the present invention is an invention of a method for manufacturing a semiconductor nanoparticle as defined in the claims, which comprises a process for allowing a functional group-containing polymer to electrostatically bind to the surface of the semiconductor nanoparticle, a process for causing functional groups of the polymer to form a crosslink via a crosslinking agent.

Specifically, the method comprises a process for arranging electron-releasing groups on the surface of a semiconductor nanoparticle by adding surface-treating material having one or more electron-releasing groups to the semiconductor nanoparticle, a process for allowing a functional group-containing polymer to electrostatically bind to the arranged electron-releasing groups, and a process for causing functional groups of the functional group-containing polymer to form a crosslink via a crosslinking agent.

As above, preferably, examples of functional groups of the functional group-containing polymer include, but are not limited to, one or more groups selected from the group consisting of -COOH, -OH, -NH₂, -SH, -OCN, -CNO, -CHO -CH=O, -CH=CH₂, and -C≡CH.

Preferably, examples of the crosslinking reaction include one or more reactions selected from the group consisting of an esterification reaction, an amidation reaction, an imidation reaction, an etherification reaction, an urethanation reaction, a sulfidation reaction, a polysulfidation reaction, a carbonate reaction, a thiolation reaction, a thioesterification reaction, and a thiourethanation reaction. A carbon-carbon double bond or carbon-carbon triple bond polymerization reaction is also effective for the formation of an organic layer as an outer shell of the semiconductor nanoparticle.

Particularly, in preferred examples, the functional group-containing polymer is polyacrylic acid and the crosslinking agent is alkylene diamine. Specifically, a semiconductor nanoparticle having further improved durability and a surface condition that is particularly preferable for bio-related applications is obtained by allowing a polymer such as polyacrylic acid to electrostatically bind to the surface of a semiconductor nanoparticle that has been coated with organic compounds or by surface reforming with the use of electric charges on the surface of the semiconductor nanoparticle, and further by allowing the polymer to form a crosslinking bond with another polymer with the use of ethylenediamine, 1-ethyl-3-(3- dimethylaminopropyl)carbodiimide (EDC), or the like.

Preferably, examples of a surface-treating material that provides electron-releasing groups to a semiconductor nanoparticle surface include at least a pure metal, a metal compound, nitrogenated compounds selected from the group consisting of ammonia, amines, ammoniums, nitriles, and isocyanates, or oxygenated compounds selected from the group consisting of alcohols, phenols, ketones, aldehydes, carboxylic acids, esters of organic or inorganic acids, ethers, acid amides, and acid anhydrides.

Thirdly, the present invention is an invention regarding semiconductor nanoparticle applications, and the invention relates to a fluorescent reagent and an optical device.

According to the present invention, semiconductor nanoparticles that are available for use in industries related to bio-applications can be synthesized. Further, reagents that are relatively safer than those used in existing methods can be used, so that a synthesis method that is performed under safe reaction conditions can be selected. Thus, semiconductor nanoparticles that are suitable for mass synthesis and the like can be produced in a more industrially adequate manner.

### BRIEF DESCRIPTION OF THE DRAWINGS

Fig. 1 shows absorbance and fluorescence spectra of a NH₃ surface reformed semiconductor nanoparticle and a semiconductor nanoparticle obtained by adding an ammonium compound to an aqueous solution containing the surface reformed semiconductor nanoparticle.
Fig. 2 shows a schematic diagram of a semiconductor nanoparticle obtained by adding an ammonium compound to a NH₃ surface reformed semiconductor nanoparticle.
Fig. 3 shows absorbance and fluorescence spectra of a semiconductor nanoparticle obtained by adding an ammonium compound to a NH₃ surface reformed semiconductor nanoparticle.
Fig. 4 shows a schematic drawing of semiconductor nanoparticles comprising a crosslink formed with polyacrylic acid and ethylenediamine.
Fig. 5 shows absorbance and fluorescence spectra of a semiconductor nanoparticle comprising a crosslink formed with polyacrylic acid and ethylenediamine.
Fig. 6 shows transmission electron microscope (TEM) pictures of a semiconductor nanoparticle comprising a crosslink formed with polyacrylic acid and ethylenediamine.

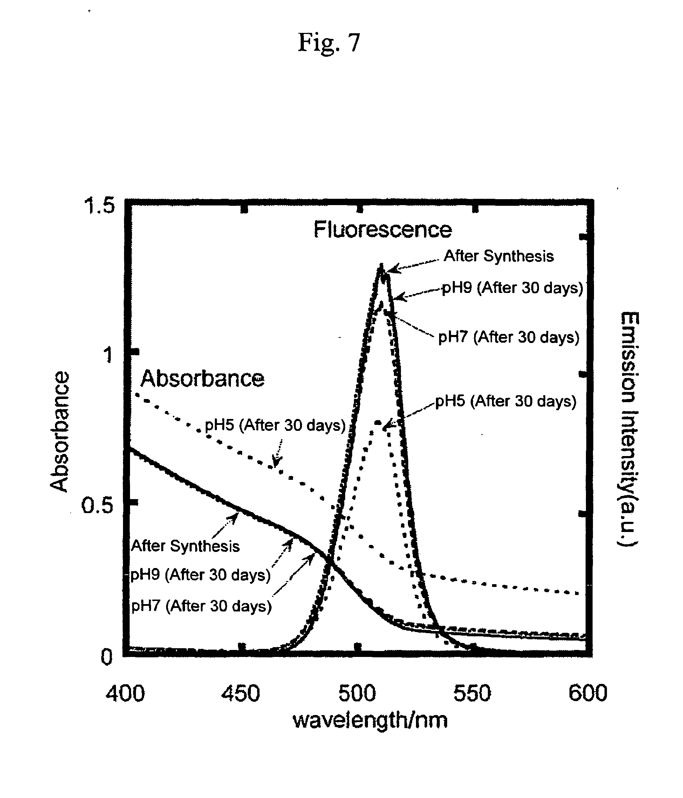

Fig. 7 shows absorbance and fluorescence spectra together with durability of a semiconductor nanoparticle synthesized according to the example 2.

### DESCRIPTION OF THE PREFERRED EMBODIMENTS

The preferred embodiments for carrying out the present invention will be described below.

### [Examples]

### (In the case of using a size selective photoetching technique)

First, 61.8 mg of sodium hexametaphosphate (0.1 mmol) and 84.4 mg of cadmium perchlorate (0.2 mmol) were added to a container filled with 1000 ml of 30°C ultrapure water, 141.960 mg of disodium hydrogenphosphate (1 mmol) was added thereto, and then this solution was stirred for 30 minutes in a container that was sealed while nitrogen was bubbled thereinto. After that, 4.96 cm⁻³ (1 atm, 25°C) of hydrogen sulfide gas was added to the aforementioned container to result in equal amounts of S²⁻ and Cd²⁺ while the container was strongly shaken, and the solution was agitated for several hours at room temperature. At this time, the color of the solution changed from optically clear colorless to optically clear yellow. Moreover, after removing unreacted hydrogen sulfide in the solution by bubbling nitrogen into the solution, oxygen bubbling was carried out for 10 minutes and 25.7 mg of methyl viologen (0.1 mmol) was added to the solution. Here, the aforementioned solution was irradiated with monochromatic light using a laser, etc. or light from a mercury vapor lamp that had passed through a color filter to control the particle size using a size selective photoetching technique. Then, after the aforementioned solution was agitated for 30 minutes while nitrogen was bubbled into the solution, 50 µl of 3-mercaptopropionic acid was added thereto and the resultant was agitated for one night under shading.

### (In the case of not using a size selective photoetching technique)

First, 61.8 mg of sodium hexametaphosphate (0.1 mmol) and 84.4 mg of cadmium perchlorate (0.2 mmol) were added to a container filled with 1000 ml of 30°C ultrapure water, 28.392 mg of disodium hydrogenphosphate (0.2 mmol) and 95.984 mg of sodium dihydrogenphosphate (0.8 mmol) were added thereto, and then this solution was stirred for 30 minutes in a container that was sealed up while nitrogen was bubbled thereinto. After that, 4.96 cm⁻³ (1 atm, 25°C) of hydrogen sulfide gas was added to the aforementioned container to result in equal amounts of S²⁻ and Cd²⁺ while the container was strongly shaken, and the solution was agitated for several hours at room temperature. Moreover, after removing unreacted hydrogen sulfide in the solution by bubbling nitrogen into the solution, 50 µl of 3-mercaptopropionic acid was added to the solution, followed by agitation for one night under shading.

1000 ml of solution prepared by either method described above was ultra-filtered and concentrated to several milliliters so as to remove methyl viologen, hexametaphosphoric acid, unreacted thiol compound, and ions, etc. dissolved upon photoetching from the aqueous solution, such that a solution containing semiconductor nanoparticles having surfaces modified with a pure thiol compound was prepared. Then, it was ultra-filtered by adding pure water and refined by repeating this process several times. Thereafter, a surface reforming treatment was performed by using the solution, which was finally concentrated to several milliliters.

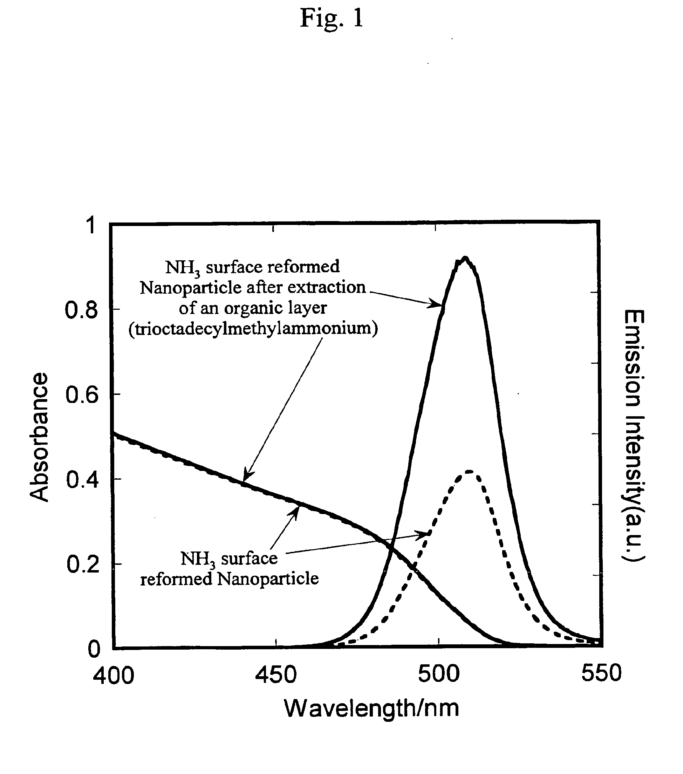

The refined thiol-modified semiconductor nanoparticle solution obtained as described above was diluted by using 0.1M NH₃ aq. so as to have an absorbance of 0.5, and surface treatment was carried out by allowing it stand for several days under irradiating fluorescent light. Accordingly, a semiconductor nanoparticle solution having high luminescence properties was obtained. The obtained solution was optically clear yellow and it had excellent luminescence properties. Fluorescence spectra from such time are shown in Fig. 1.

A mixed solution made by adding tridodecylmethylammonium chloride to an organic solvent such as hexane to a concentration of 1 mg/ml with respect to the solvent was added to the aforementioned surface reformed semiconductor nanoparticle solution in an amount such that it accounted for 1/10 of the amount of the solution. Or, alternatively, a mixed solution made by adding trioctadecylmethylammonium bromide to an organic solvent to a concentration of 2 mg/ml with respect to the solvent was added to the surface reformed semiconductor nanoparticle solution in an amount such that it accounted for 1/10 of the amount of the solution, and methanol was added thereto in an amount such that it accounted for 1/5 of the amount of the solution. Either one of these resulting solutions was strongly agitated for a certain time. As a result, it could be confirmed that the optically clear yellow part was transferred from the aqueous phase to the organic phase. Then, after performing a centrifugal separation, the aqueous phase and the organic phase were separated. The aforementioned recovered organic phase was diluted by adding an organic solvent such as hexane so as to result in the same absorbance as that of the aforementioned aqueous solution before transfer. The semiconductor nanoparticles transferred to the organic phase still maintained high luminescence properties. Fluorescence spectra from such time are shown in Fig. 1.

### [Example 1 not in accordance with the present invention]

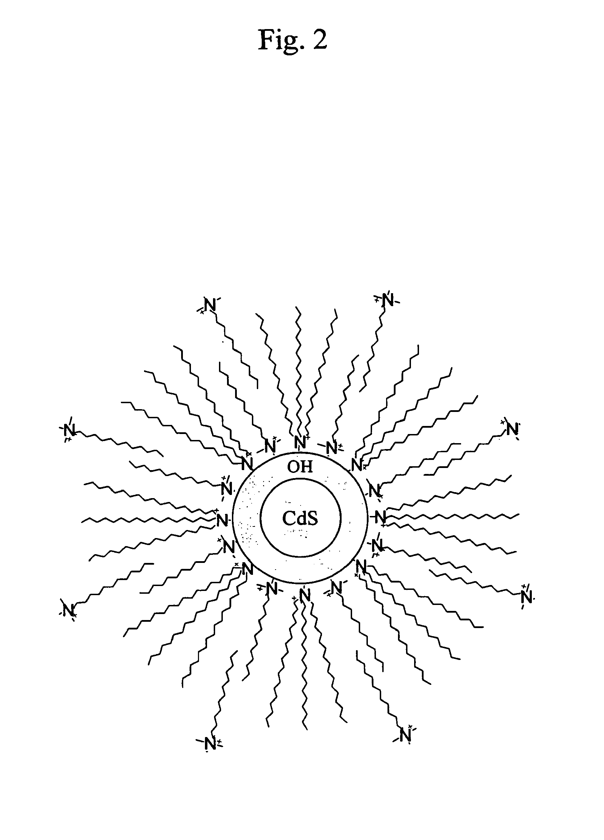

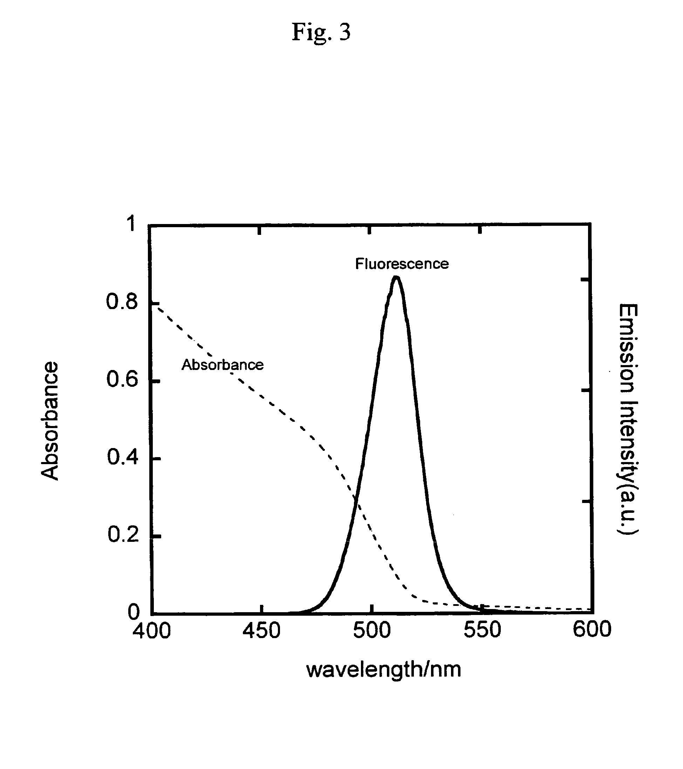

Nanoparticles perfectly dispersed into the organic solvent obtained as mentioned above were coated with amphipathic molecules. 10 ml of a solution in which nanoparticles had perfectly dispersed into the aforementioned organic solvent was put into a container such as a stoppered test tube or an eggplant shaped flask, etc. and was made to assume a membranous form on the inner wall of the container by evaporation. Then, the particles were dissolved again by adding 2 ml of solution in which dodecyltrimethylammonium chloride was dissolved in chloroform to a concentration of 5 mM, and the resulting solution was made to assume a membranous form again on the inner wall of the container by evaporation. Moreover, after removing residual chloroform by heating the container at 90°C, the particles were dissolved again by adding 2 ml of methanol. Then, methanol was removed by adding 10 ml of ultrapure water, followed by agitation for some time during heating to 90°C. Finally, an optically clear yellow solution could be obtained by performing centrifugal separation so as to remove the precipitation. A schematic drawing and optical spectra at this time are shown in Figs. 2 and 3, respectively.

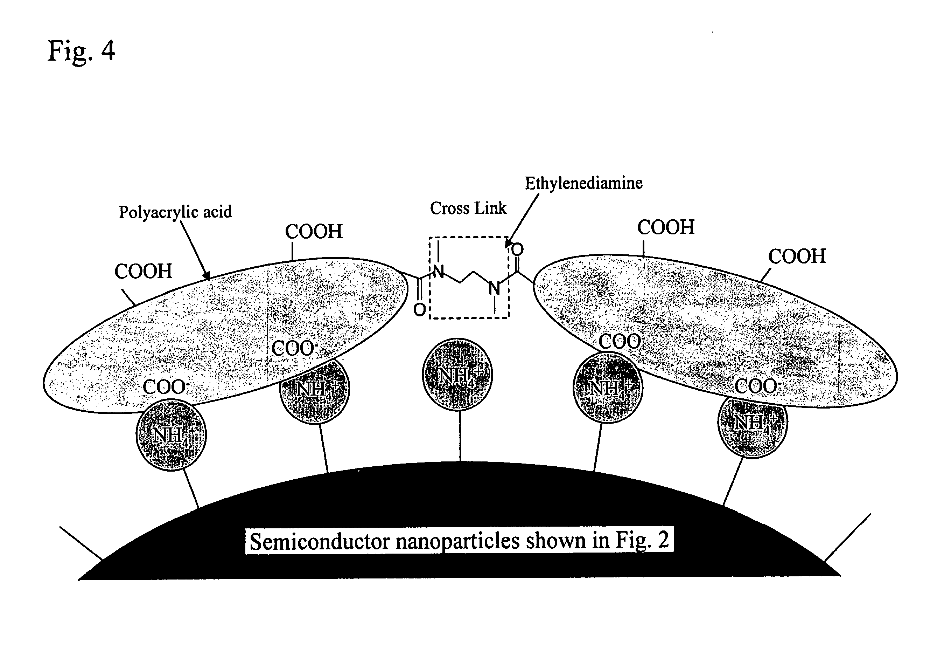

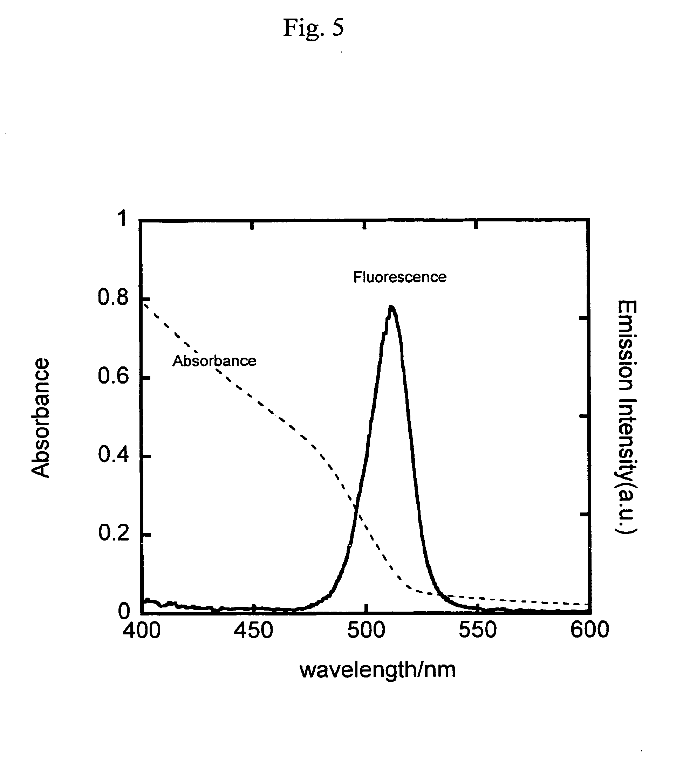

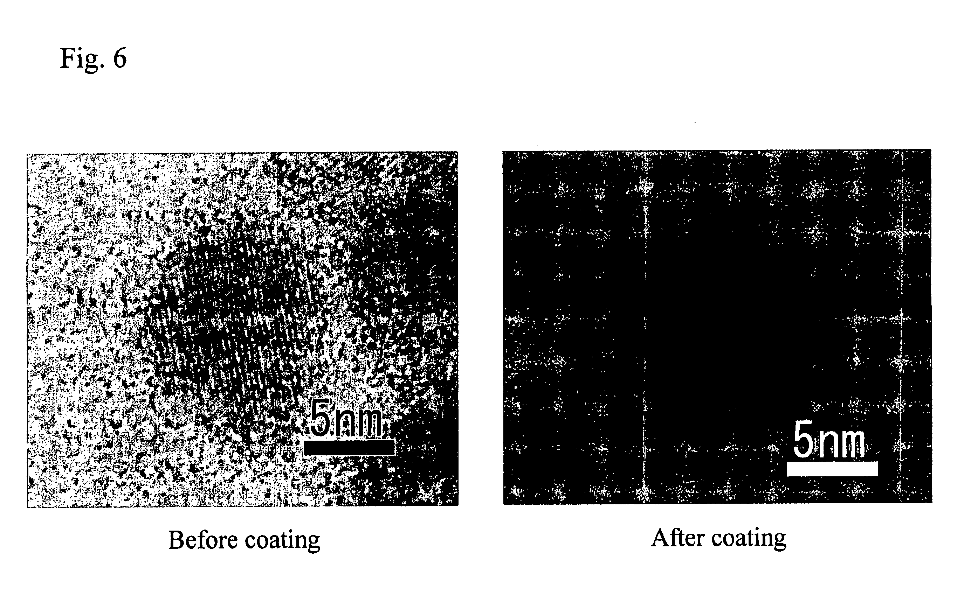

Polyacrylic acid (average molecular weight: 5000) and ethylenediamine were added to the obtained optically clear yellow solution to concentrations of 0.1 mM and 1.5 mM therein, respectively, followed by agitation for some time. Further, hydrochloric acid 1-ethyl-3-(3-dimethylaminopropyl)carbodiimide was added thereto to a concentration of 10 mM therein, followed by agitation for several days. Thereafter, the resultant was ultra-filtered so as to be refined. A schematic drawing, fluorescence spectra, and transmission electron microscope (TEM) pictures from such time are shown in Figs. 4, 5, and 6, respectively.

Particle having further improved durability can be synthesized by arbitrarily selecting the types of surfactants and crosslinking agents for use, the types of solvents and methods for replacing the solvents, temperature conditions, etc.

### [Example 2]

CdS nanoparticles perfectly dispersed into the organic solvent obtained as mentioned above (10 ml) were put into an eggplant shaped flask, and then hexane was removed by evaporation. 2 ml of chloroform was added and octadecylamine was added thereto to a concentration of 0.5 mM, and then, they were dissolved using ultrasonic wave. Further, the chloroform was removed by evaporation, and 2 ml of THF was added thereto for dissolving the nanoparticles again. Here, ultrasonic treatment may be used. Then, 10 ml of ultrapure water and a stirring bar were put into an eggplant shaped flask, and CdS nanoparticles dissolved in THF were rapidly poured thereto while agitated by the stirrer. The solution was dissolved by ultrasonic treatment, etc, only THF was removed by evaporation for obtaining a water solution, and then, the water solution was put into a centrifuge tube for removing precipitation by centrifugation. Thereby, water-soluble nanoparticles with amino groups arranged on the surfaces thereof were obtained.

10 ml of the obtained solution and a stirring bar were put into an eggplant shaped flask, and 50 µl of 0.2 M-polyacrylic acid adjusted to pH 7 was added thereto, and agitated by the stirrer for one hour. 1 µl of ethylenediamine is added thereto and agitated by the stirrer for ten minutes, and then, 19.2 mg of WSC was added and further agitated by the stirrer overnight. In this case, the polyacrilic acids binding to the surfaces of the particles are crosslinked by ethylenediamine. When amine is used as amphipathic molecules to make the nanoparticles water-soluble, this amine also binds to the polyacrilic acids. This solution was put into the centrifuge tube for removing precipitation by centrifugation, and then, flow cleaning was carried out using ultrafiltration equipment of 50 ml cells. Although precipitation was produced under an acidic region, the obtained particles continued to maintain the same fluorescence property for more than one month under regions of pH 5 to 9 in pure water.

Each of the nanoparticles obtained according to either the example 1 or 2 has a carboxyl group exposed on the surface thereof. Such configuration is preferable for staining and labeling of biopolymers.

As described above, the present invention is not especially limited with respect to the material of particles, the types of surfactants and crosslinking agents for use, the types of polymers to be electrostatically bound, the types of solvents and methods for replacing the solvents, concentration and temperature conditions, etc. Further, characteristic improvements in dispersibility and durability can be realized by arbitrarily selecting these conditions, and the surface design of varying the types of functional groups to be exposed to the surface or the like can be flexibly performed. Furthermore, with the use of the method of the present invention, semiconductor particles having an entirely positively charged surface condition and those having a negatively charged surface condition can alternately be laminated on each other, so that the improvement of durability of the particles can be attempted. In addition, crosslinking using ethylenediamine was carried out in the Examples, while polymers used may contain a portion capable of being crosslinked. Further, in the present invention, particle size control is not particularly carried out. It is also possible to obtain fine particle sizes using an ultrasonic homogenizer, etc.; however, any particle size can be appropriate.

According to the present invention, it becomes possible to easily synthesize semiconductor nanoparticles that have high luminescence properties and excellent chemical stability. The semiconductor nanoparticles of the present invention can be used for fluorescent reagents and optical devices, etc. by utilizing such high luminescence properties.

## Claims

1. A semiconductor nanoparticle, which is modified with a functional group-containing polymer that electrostatically binds to the surface of said semiconductor nanoparticle via a semiconductor nanoparticle-coating compound which is octadecylamine.

2. The semiconductor nanoparticle according to claim 1, wherein electron-releasing groups are arranged on the surface of said semiconductor nanoparticle and said polymer electrostatically binds to the outside of said electron-releasing groups.

3. The semiconductor nanoparticle according to claim 1 or claim 2, wherein said functional group-containing polymer forms a crosslinking bond via a crosslinking agent.

4. The semiconductor nanoparticle according to any one of claims 1 to 3, comprising said functional group-containing polymer whose functional group is one or more functional groups selected from the group consisting of -COOH, -OH, -NH₂, -SH, -OCN, -CNO, -CHO, -CH=O, -CH=CH₂, and -C≡CH.

5. The semiconductor nanoparticle according to claim 3, wherein said crosslinking bond is one or more bonds selected from the group consisting of an ester bond, an amide bond, an imide bond, an ether bond, a urethane bond, a sulfide bond, a polysulfide bond, a carbonate bond, a thiol bond, a thioester bond, and a thiourethane bond.

6. The semiconductor nanoparticle according to claim 3, wherein said crosslinking bond results from carbon-carbon double bond or carbon-carbon triple bond polymerization.

7. The semiconductor nanoparticle according to any one of claims 1 to 6, wherein said functional group-containing polymer is polyacrylic acid and said crosslinking agent is alkylene diamine.

8. The semiconductor nanoparticle according to claim 2, wherein said electron-releasing group is at least one electron-releasing group selected from the group consisting of -OR, -OCH₂R, -OCOCH₂R, -NHR, -N(CH₂R)₂, -NHCOCH₂R, -CH₂R, and -C₆H₄R, where R is hydrogen, a substituted hydrocarbon group, or an unsubstituted hydrocarbon group.

9. The semiconductor nanoparticle according to any one of claims 1 to 8, comprising a semiconductor nanoparticle whose material is one or more materials selected from the group consisting of ZnO, ZnS, ZnSe, ZnTe, CdO, CdS, CdMnS, CdSe, CdMnSe, CdTe, CdMnTe, HgS, HgSe, HgTe, InP, InAs, InSb, InN, GaN, GaP, GaAs, GaSb, TiO₂, WO₃, PbS, PbSe, MgTe, AlAs, AlP, AlSb, AlS, Ge, and Si, or a semiconductor nanoparticle having a multilayer structure consisting of a core portion and a shell portion that are made of one or more members of said group.

10. The semiconductor nanoparticle according to any one of claims 1 to 9, wherein the particle size of said semiconductor nanoparticle exhibits a deviation of less than 10 % rms in diameter, thereby achieving monodispersion.

11. The semiconductor nanoparticle according to any one of claims 1 to 10, wherein said semiconductor nanoparticle emits light in a narrow spectrum range of less than 60 nm in terms of full width at half maximum (FWHM) upon being irradiated with excitation light.

12. A method of manufacturing a semiconductor nanoparticle comprising:
a process for arranging electron-releasing groups on the surface of a semiconductor nanoparticle by adding surface-treating material that provides one or more electron-releasing groups to said semiconductor nanoparticle;
a process for coating said semiconductor nanoparticle, on which the electron-releasing groups are arranged, with a semiconductor nanoparticle-coating compound which is octadecylamine;
a process for allowing a functional group-containing polymer to electrostatically bind to the surface of said semiconductor nanoparticle via said octadecylamine; and
a process for causing functional groups of said polymer to form a crosslink via a crosslinking agent.

13. The method of manufacturing a semiconductor nanoparticle according to claim 12, comprising said functional group-containing polymer whose functional group is one or more functional groups selected from the group consisting of -COOH, -OH, -NH₂, -SH, -OCN, -CNO, -CHO, -CH=O, -CH=CH₂, and -C≡CH.

14. The method of manufacturing a semiconductor nanoparticle according to any one of claims 12 or 13, wherein said crosslink is formed by one or more reactions selected from the group consisting of an esterification reaction, an amidation reaction, an imidation reaction, an etherification reaction, a urethanation reaction, a sulfidation reaction, a polysulfidation reaction, a carbonate reaction, a thiolation reaction, a thioesterification reaction, and a thiourethanation reaction.

15. The method of manufacturing a semiconductor nanoparticle according to any one of claims 12 or 13, wherein said crosslink is formed by a carbon-carbon double bond or carbon-carbon triple bond polymerization reaction.

16. The method of manufacturing a semiconductor nanoparticle according to any one of claims 12 to 15, wherein said functional group-containing polymer is polyacrylic acid and said crosslinking agent is alkylene diamine.

17. The method of manufacturing a semiconductor nanoparticle according to any one of claims 12 to 16, wherein said surface-treating material that provides electron-releasing groups to the semiconductor nanoparticle is at least a pure metal, or a metal compound, nitrogenated compounds selected from the group consisting of ammonia, amines, ammoniums, nitriles, and isocyanates, or oxygenated compounds selected from the group consisting of alcohols, phenols, ketones, aldehydes, carboxylic acids, esters of organic or inorganic acids, ethers, acid amides, and acid anhydrides.

18. The method of manufacturing a semiconductor nanoparticle according to any one of claims 12 to 17, comprising a semiconductor nanoparticle whose material is one or more materials selected from the group consisting of ZnO, ZnS, ZnSe, ZnTe, CdO, CdS, CdMnS, CdSe, CdMnSe, CdTe, CdMnTe, HgS, HgSe, HgTe, InP, InAs, InSb, InN, GaN, GaP, GaAs, GaSb, TiO₂, WO₃, PbS, PbSe, MgTe, AlAs, AlP, AlSb, AlS, Ge, and Si, or a semiconductor nanoparticle having a multilayer structure consisting of a core portion and a shell portion that are made of one or more members of said group.

19. A fluorescent reagent comprising the semiconductor nanoparticle according to any one of claims 1 to 12.

20. An optical device comprising the semiconductor nanoparticle according to any one of claims 1 to 12.

## Patentansprüche

1. Halbleiter-Nanopartikel, welches mit einem eine funktionale Gruppe enthaltenden Polymer modifiziert ist, welches mit der Oberfläche des Halbleiter-Nanopartikels über eine Halbleiter-Nanopartikel-Beschichtungsverbindung elektrostatisch gebunden ist, die aus Oktadecylamin besteht.

2. Halbleiter-Nanopartikel nach Anspruch 1, bei dem auf der Oberfläche des Halbleiter-Nanopartikels elektronen-abgebende Gruppen angeordnet sind und das Polymer elektrostatisch mit den äußeren der elektronen-abgebenden Gruppen bindet.

3. Halbleiter-Nanopartikel nach Anspruch 1 oder Anspruch 2, bei dem das eine funktionale Gruppe enthaltende Polymer eine Vernetzungs-Verbindung über ein Vernetzungsmittel bildet.

4. Halbleiter-Nanopartikel nach einem der Ansprüche 1 bis 3, welches das genannte eine funktionale Gruppe enthaltende Polymer umfasst, dessen funktionale Gruppe durch eine oder mehrere der folgenden funktionalen Gruppen gebildet wird: -COOH, -OH, -NH₂, -SH, -OCN, -CNO, -CHO, -CH=O, -CH=CH₂ und -C≡CH.

5. Halbleiter-Nanopartikel nach Anspruch 3, bei dem die Vemetzungs-Verbindung durch eine oder mehrere der folgenden Verbindungen gebildet wird: Ester-Verbind-ung, Amid-Verbindung, Imid-Verbindung, Ether-Verbindung, Urethan-Verbindung, Sulfid-Verbindung, Polysulfid-Verbindung, Carbonat-Verbindung, Thiol-Verbindung, Thioester-Verbindung, oder Thiourethan-Verbindung.

6. Halbleiter-Nanopartikel nach Anspruch 3, bei dem die Vemetzungsverbindung aus einer Polymerisation einer Kohlenstoff-Kohlenstoff-Doppelbindung oder einer Kohlenstoff-Kohlenstoff-Dreifachbindung resultiert.

7. Halbleiter-Nanopartikel nach einem der Ansprüche 1 bis 6, bei der das eine funktionale Gruppe enthaltende Polymer durch Polyacrylsäure gebildet wird und das Vernetzungsmittel durch Alkylen-Diamin gebildet wird.

8. Halbleiter-Nanopartikel nach Anspruch 2, bei dem die elektronen-abgebende Gruppe durch mindestens eine der folgenden elektronen-abgebenden Gruppen gebildet wird: -OR, -OCH₂R₂, -OCOCH₂R, -NHR, -N(CH₂R)₂, -NHCOCH₂R, -CH₂R, und C₆H₄R, wobei R durch Wasserstoff, eine substituierte Kohlenwasserstoffgruppe oder eine nicht-substituierte Kohlenwasserstoffgruppe gebildet wird.

9. Halbleiter-Nanopartikel nach einem der Ansprüche 1 bis 8, umfassend ein Halbleiter-Nanopartikel, dessen Material durch eines oder mehrere der folgenden Materialen gebildet wird: ZnO, ZnS, ZnSe, ZnTe, CdO, CdS, CdMnS, CdSe, CdMnSe, CdTe, CdMnTe, HgS, HgSe, HgTe, InP, InAs, InSb, InN, GaN, GaP, GaAs, GaSb, TiO₂, WO₃, PbS, PbSe, MgTe, AlAs, AlP, AlSb, AlS, Ge und Si, oder ein Halbleiter-Nanopartikel umfassend, welches eine Mehrschichtstruktur aufweist, die aus einem Kernabschnitt und einem Hüllenabschnitt besteht, die aus einem oder mehreren der oben genannten Materialien bestehen.

10. Halbleiter-Nanopartikel nach einem der Ansprüche 1 bis 9, bei dem die Partikelgröße des Halbleiter-Nanopartikels eine Abweichung bezüglich des Durchmessers mit einem Effektivwert von weniger als 10% aufweist und **dadurch** eine Monodispersion erreicht.

11. Halbleiter-Nanopartikel nach einem der Ansprüche 1 bis 10, bei dem das Halbleiter-Nanopartikel-Licht in einem engen Spektralbereich mit einer Halbwertsbreite von weniger als 60nm (FWHM) emittiert, wenn es mit Anregungslicht bestrahlt wird.

12. Verfahren zum Herstellen eines Halbleiter-Nanopartikels, das folgendes umfasst:
einen Prozess zum Anordnen von elektronen-abgebenden Gruppen auf der Oberfläche
eines Halbleiter-Nanopartikels durch Zufügen eines Oberflächen-Behandlungsmaterials, welches eine oder mehrere elektronen-abgebende Gruppen auf dem Halbleiter-Nanopartikel bereitstellt;
einen Prozess zum Beschichten des Halbleiter-Nanopartikels, auf dem die elektronen-abgebenden Gruppen angeordnet sind, mit einer Nanopartikel-Beschichtungsverbindung, bei der es sich um Oktadecylamin handelt;
einen Prozess, bei dem es einem eine funktionale Gruppe enthaltenden Polymer gestattet wird, elektrostatisch mit der Oberfläche des Halbleiter-Nanopartikels über das Oktadecylamin zu binden; und
einen Prozess, in dem veranlasst wird, dass die funktionalen Gruppen des Polymers über ein Vernetzungsmittel eine Vernetzung bilden.

13. Verfahren zum Herstellen eines Halbleiter-Nanopartikels nach Anspruch 12, welches das genannte eine funktionale Gruppe enthaltende Polymer umfasst, wobei die funktionale Gruppe durch eine oder mehrere der folgenden Gruppen gebildet wird: -COOH, -OH, -NH₂, -SH, -OCN, -CNO, -CHO, -CH=O, -CH=CH₂ und -C≡CH.

14. Verfahren zum Herstellen eines Halbleiter-Nanopartikels nach einem der Ansprüche 12 oder 13, bei dem die Vernetzung durch eine oder mehrere der folgenden Reaktionen gebildet wird: eine Veresterungs-Reaktion, eine Amidierungs-Reaktion, eine Imidierungs-Reaktion, eine Veretherungs-Reaktion, eine Urethanisierung-Reaktion, eine Sulfidierungs-Reaktion, eine Polysulfidierungs-Reaktion, eine Carbonat-Reaktion, eine Thiolisierungs-Reaktion, eine Thioesterisierungs-Reaktion und eine Thiourethanisierungsreaktion.

15. Verfahren zum Herstellen eines Halbleiter-Nanopartikels nach einem der Ansprüche 12 oder 13, bei dem die Vernetzung durch die Polymerisations-Reaktion einer Kohlenstoff-Kohlenstoff-Doppelbindung oder einer Kohlenstoff-Kohlenstoff-Dreifachbindung gebildet wird.

16. Verfahren zum Herstellen eines Halbleiter-Nanopartikels nach einem der Ansprüche 12 bis 15, bei dem das eine funktionale Gruppe enthaltende Polymer durch Polyacrylsäure gebildet wird und das Vernetzungsmittel durch Alkylen-Diamin gebildet wird.

17. Verfahren zum Herstellen eines Halbleiter-Nanopartikels nach einem der Ansprüche 12 bis 16, bei dem das Oberflächen-Behandlungsmaterial, welches die elektronen-abgebenden Gruppen für das Halbleiter-Nanopartikel bereitstellt, mindestens eines der folgenden Materialien umfasst: ein reines Metall oder eine Metallverbindung, nitrogenisierte Verbindungen aus der folgenden Gruppe: Amoniak, Amine, Amoniume, Nitride und Isocyanate, oder oxygenierte Verbindungen aus der folgenden Gruppe: Alkohole, Phenole, Ketone, Aldehyde, Carboxylsäuren, Ester aus organischen oder anorganischen Säuren, Ether, Säureamide und Säureanhydride.

18. Verfahren zum Herstellen eines Halbleiter-Nanopartikels nach einem der Ansprüche 12 bis 17, umfassend ein Halbleiter-Nanopartikel, dessen Material durch eines oder mehrere der folgenden Materialen gebildet wird: ZnO, ZnS, ZnSe, ZnTe, CdO, CdS, CdMnS, CdSe, CdMnSe, CdTe, CdMnTe, HgS, HgSe, HgTe, InP, InAs, InSb, InN, GaN, GaP, GaAs, GaSb, TiO₂, WO₃, PbS, PbSe, MgTe, AlAs, AlP, AlSb, AlS, Ge und Si, oder ein Halbleiter-Nanopartikel mit einer mehrschichtigen Struktur, bestehend aus einem Kernabschnitt und einem Hüllenabschnitt, welche aus einem oder mehreren der genannten Materialien bestehen.

19. Fluoreszierendes Reagenz, welches ein Halbleiter-Nanopartikel nach einem der Ansprüche 1 bis 12 umfasst.

20. Optische Vorrichtung, umfassend ein Halbleiter-Nanopartikel nach einem der Ansprüche 1 bis 12.

## Revendications

1. Particule semiconductrice, modifiée avec un polymère contenant un groupe fonctionnel qui se lie de façon électrostatique à la surface de la nanoparticule semiconductrice par l'intermédiaire d'un composé de revêtement de nanoparticule semiconductrice qui est de l'octadécylamine.

2. Nanoparticule semiconductrice selon la revendiçation 1, dans laquelle des groupes à libération d'électron sont agencés sur la surface de la nanoparticule semiconductrice et ledit polymère se lie de façon électrostatique à l'extérieur des groupes à libération d'électron.

3. Nanoparticule semiconductrice selon la revendiçation 1 ou 2, dans laquelle le polymère contenant un groupe fonctionnel forme une liaison de réticulation par l'intermédiaire d'un agent de réticulation.

4. Nanoparticule semiconductrice selon l'une quelconque des revendications 1 à 3, comprenant le polymère contenant un groupe fonctionnel et dont le groupe fonctionnel est un ou plusieurs groupes fonctionnels choisis dans le groupe comprenant -COOH, -OH, -NH₂, -SH, -OCN, -CNO, -CHO, -CH=O,-CH=CH₂ et -C≡CH.

5. Nanoparticule semiconductrice selon la revendiçation 3, dans laquelle la liaison de réticulation est constituée d'une ou plusieurs liaisons choisies dans le groupe comprenant une liaison ester, une liaison amide, une liaison imide, une liaison éther, une liaison uréthane, une liaison sulfure, une liaison polysulfure, une liaison carbonate, une liaison thiol, une liaison thio-ester, et une liaison thio-uréthane.

6. Nanoparticule semiconductrice selon la revendication 3, dans laquelle la liaison de réticulation résulte d'une polymérisation à liaison double carbone-carbone ou à liaison triple carbone-carbone.

7. Nanoparticule semiconductrice selon l'une quelconque des revendications 1 à 6, dans laquelle le polymère contenant un groupe fonctionnel est de l'acide polyacrylique et l'agent de réticulation est une diamine non saturée.

8. Nanoparticule semiconductrice selon la revendiçation 2, dans laquelle le groupe à libération d'électron est au moins un groupe à libération d'électron choisis dans le groupe comprenant -OR, -OCH₂R, -OCOCH₂R, -NHR, -N(CH₂R)₂, -NHCOCH₂R, -CH₂R, et -C₆H₄R, où R est de l'hydrogène, un groupe hydrocarbure substitué ou un groupe hydrocarbure non substitué.

9. Nanoparticule semiconductrice selon l'une quelconque des revendications 1 à 8, comprenant une nanoparticule semiconductrice dont le matériau est un ou plusieurs matériaux choisis dans le groupe comprenant ZnO, ZnS, ZnSe, ZnTe, CdO, CdS, CdMnS, CdSe, CdMnSe, CdTe, CdMnTe, HgS, HgSe, HgTe, InP, InAs, InSb, InN, GaN, GaP, GaAS, GaSb, TiO₂, WO₃, PbS, PbSe, MgTe, AlAs, AlP, AlSb, AlS, Ge, et Si, ou bien une nanoparticule semiconductrice ayant une structure multicouche constituée d'une partie de coeur et d'une partie de coque qui sont faites d'un ou plusieurs des éléments dudit groupe.

10. Nanoparticule semiconductrice selon l'une quelconque des revendications 1 à 9, dans laquelle la taille de la nanoparticule semiconductrice présente un écart quadratique moyen (rms) en diamètre inférieur à 10 %, réalisant ainsi une mono-dispersion.

11. Nanoparticule semiconductrice selon l'une quelconque des revendications 1 à 10, dans laquelle la nanoparticule semiconductrice émet de la lumière dans une plage spectrale étroite inférieure à 60 nm en tant que largeur totale à mi-hauteur (FWHM) lorsqu'elle est irradiée avec une lumière d'excitation.

12. Procédé de fabrication d'une nanoparticule semiconductrice comprenant :
un processus pour agencer des groupes à libération d'électron sur la surface d'une nanoparticule semiconductrice en ajoutant un matériau de traitement de surface qui fournit un ou plusieurs groupes à libération d'électron à la nanoparticule semiconductrice ;
un processus pour revêtir la nanoparticule semiconductrice, sur laquelle les groupes à libération d'électron sont agencés, d'un composé de revêtement de nanoparticule semiconductrice qui est de l'octadécylamine ;
un processus pour permettre à un polymère contenant un groupe fonctionnel de se lier de façon électrostatique à la surface de la nanoparticule semiconductrice par l'intermédiaire de l'octadécylamine ; et
un processus pour faire en sorte que des groupes fonctionnels du polymère forment une liaison de réticulation par l'intermédiaire d'un agent de réticulation.

13. Procédé de fabrication d'une nanoparticule semiconductrice selon la revendication 12, comprenant le polymère contenant un groupe fonctionnel et dont le groupe fonctionnel est un ou plusieurs groupes fonctionnels choisis dans le groupe comprenant -COOH, -OH, -NH₂, -SH, -OCN, -CNO, -CHO, -CH=O,-CH=CH₂, et -C≡H.

14. Procédé de fabrication d'une nanoparticule semiconductrice selon la revendication 12 ou 13, dans lequel la liaison de réticulation est formée par une ou plusieurs réactions choisies dans le groupe comprenant une réaction d'estérification, une réaction d'amidation, une réaction d'imidation, une réaction d'éthérification, une réaction d'uréthanation, une réaction de sulfuration, une réaction de polysulfuration, une réaction de carbonatation, une réaction de thiolation, une réaction de thio-esterification, et une réaction de thio-uréthanation.

15. Procédé de fabrication d'une nanoparticule semiconductrice selon l'une quelconque des revendications 12 ou 13, dans lequel la liaison de réticulation est formée par une réaction de polymérisation à liaison double carbone-carbone ou à liaison triple carbone-carbone.

16. Procédé de fabrication d'une nanoparticule semiconductrice selon l'une quelconque des revendications 12 à 15, dans lequel le polymère contenant un groupe fonctionnel est de l'acide polyacrylique et l'agent de réticulation est une diamine non saturée.

17. Procédé de fabrication d'une nanoparticule semiconductrice selon l'une quelconque des revendications 12 à 16, dans lequel le matériau de traitement de surface qui fournit des groupes à libération d'électron à la nanoparticule semiconductrice est au moins un métal pur, ou un métal composé, des composés azotés choisis dans le groupe comprenant de l'ammoniac, des amines, des composés d'ammonium, des nitriles, des isocyanates, ou des composés oxygénés choisis dans le groupe comprenant des alcools, des phénols, des acétones, des aldéhydes, des acides carboxyliques, des esters d'acides organiques ou inorganiques, des éthers, des amides d'acides et des anhydrides d'acides.

18. Procédé de fabrication d'une nanoparticule semiconductrice selon l'une quelconque des revendications 12 à 17, comprenant une nanoparticule semiconductrice dont le matériau est un ou plusieurs matériaux choisis dans le groupe comprenant ZnO, ZnS, ZnSe, ZnTe, CdO, CdS, CdMnS, CdSe, CdMnSe, CdTe, CdMnTe, HgS, HgSe, HgTe, InP, InAs, InSb, InN, GaN, GaP, GaAS, GaSb, TiO₂, WO₃, PbS, PbSe, MgTe, AlAs, AlP, AlSb, AlS, Ge, et Si, ou bien une nanoparticule semiconductrice ayant une structure multicouche constituée d'une partie de coeur et d'une partie de coque qui sont faites d'un ou plusieurs des éléments dudit groupe.

19. Réactif fluorescent comprenant la nanoparticule semiconductrice selon l'une quelconque des revendications 1 à 12.

20. Dispositif optique comprenant la nanoparticule semiconductrice selon l'une quelconque des revendications 1 à 12.
